# EUROPEAN PATENT APPLICATION

(11) **EP 4 317 448 A1**
(43) Date of publication of application: **07.02.2024**
(21) Application number: 22777578.0
(22) Date of filing: 18.03.2022
(51) Int. Cl.: C12P 19/12, A23K 20/158, A23K 20/163, A23L 2/52, A23L 29/10, A23L 33/125, A23L 33/145, A61K 8/60, A61K 47/26, A61Q 19/10, C05G 3/50, C07H 15/04, C07H 15/08, C09K 23/52, C11D 1/06, C11D 1/68, C11D 3/22

(54) **NOVEL SOPHOROLIPID DERIVATIVE**

(30) Priority: 31.03.2021 JP 2021059097; 01.10.2021 JP 2021162645
(71) Applicant: Allied Carbon Solutions Co., Ltd., Shizuoka 410-0873 (JP); National Institute Of Advanced Industrial Science and Technology, Chiyoda-ku Tokyo 100-8921 (JP)
(72) Inventor: FUKUOKA, Tokuma, Tsukuba-shi, Ibaraki 305-8560 (JP); MORITA, Tomotake, Tsukuba-shi, Ibaraki 305-8560 (JP); KOBAYASHI, Yosuke, Numazu-shi, Shizuoka 410-0873 (JP); HIROTA, Makoto, Numazu-shi, Shizuoka 410-0873 (JP); YASHIRO, Makoto, Numazu-shi, Shizuoka 410-0873 (JP); HIRAYAMA, Shuji, Numazu-shi, Shizuoka 410-0873 (JP); SHIBA, Shunji, Numazu-shi, Shizuoka 410-0873 (JP); YAMAGATA, Yosuke, Numazu-shi, Shizuoka 410-0873 (JP)
(74) Representative: TBK
(86) International application number: PCT/JP2022/012536
(87) International publication number: WO 2022/210011

(57) **Abstract**

There is provided a novel sophorolipid derivative having high water solubility and surface activity. A plurality of sophorolipid derivatives having a novel structure is isolated and purified from a cultured product of sophorolipid producing microorganism

(*Starmerella bombicola,* etc.)

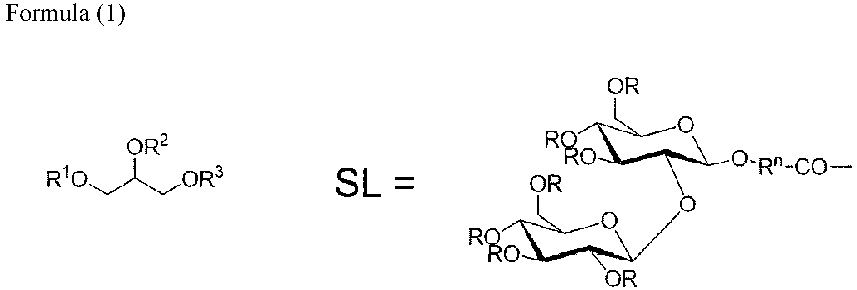

(wherein, R¹ to R³ are each independently hydrogen, a fatty acid ester having 2 to 22 carbon atoms, or the above SL group, provided that at least one of R¹ to R³ is the SL group in which R are the same or different and each represent hydrogen or an acetyl group and Rⁿ is a linear or branched alkyl or alkenyl group having 13 to 21 carbon atoms).

## Description

### BACKGROUND OF THE INVENTION

### Field of the Invention

The present invention relates to a novel sophorolipid derivative and a composition containing the derivative.

### Background Art

Among sugar-based surfactants having a hydrophilic group composed of sugar, sugar-based biosurfactants which are natural surfactants derived from microorganisms and have high safety are known as excellent surfactants. Of them, a sophorolipid is a glycolipid, has an amphipathic structure and therefore has a strong surface active action, and has high biodegradability and safety, so that applications of it are being developed as a leading biosurfactant.

*Starmerella bombicola,* a basidiomycetous yeast, is representative of a sophorolipid-producing yeast and its productivity of biosurfactants reaches even to 400 g or more per L of a culture solution, so that it is used for the production on a commercial basis.

A sophorolipid is a glycolipid having a lactonic (LSL) molecular structure represented by the following formula (3) and formed by the ether linkage of a hydroxy fatty acid to the 1 position of a sophorose which is a disaccharide formed by the (2→1) ether linkage of glucose units and/or an acidic (ASL) molecular structure represented by the following formula (4). A microbial product has one of them or a mixture of two or more of them.

A lactonic (nonionic) sophorolipid and an acidic (anionic) one are greatly different from each other in the properties as a surfactant and techniques for obtaining a mixture of them different in composition, a method of separately preparing them, or the like is being developed to increase the variety of the structure or function of sophorolipid products.

For example, it is reported that a sophorolipid produced by the aforesaid *Starmerella bombicola* is generally obtained as a 6 to 8 : 2 to 4 mixture of a lactonic form and an acidic form and the lactonic form, a main component, shows excellent surface tension reducing ability at a low concentration compared to an acidic form (Non-Patent Document 1) and in addition, shows high antibacterial activity (Non-Patent Document 2).

On the other hand, there is reported a detergent containing a chemically-stable highly-pure acidic sophorolipid obtained by the ring-opening of a lactone ring by hydrolysis (Patent Document 1), and there is also reported a method of selectively producing only an acidic sophorolipid by culturing *Candida floricola* as a producing microorganism (Patent Document 2).

And there are reported a sophorolipid derivative obtained by modifying a plurality of functional groups thereof and also a derivative having a novel structure such as polymeric sophorolipid which is different in classification from the lactonic form and acidic form (Patent Document 3 and Non-Patent Document 3).

Further, it has also been reported that by culturing *Candida batistae* as a producing strain, a sophorolipid with different structures of fatty acid moieties that bind to sophorose can be obtained (Non-Patent Document 4).

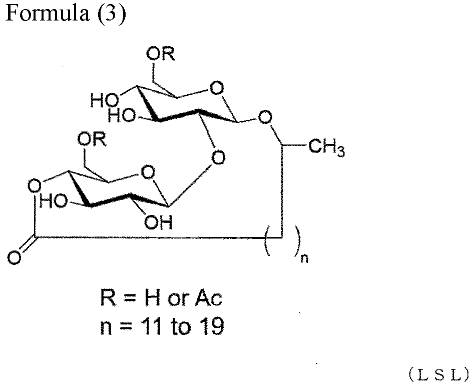

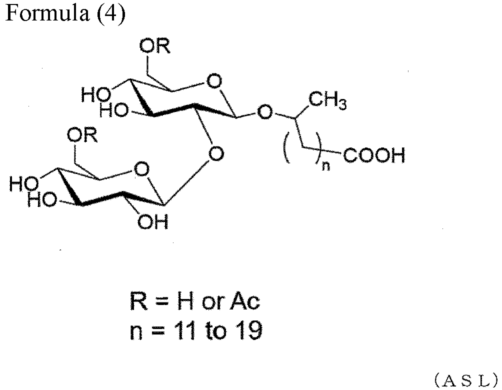

[Patent Document 1] Japanese Patent Application Laid-Open No. 2006-70231
[Patent Document 2] Japanese Patent Application Laid-Open No. 2008-247845
[Patent Document 3] WO2015/020114

[Non-Patent Document 1] Journal of Oleo Science (2013) Vo. 62, p. 857-864
[Non-Patent Document 2] Journal of Microbiology and Biotechnology (2002) Vol. 12, p. 235-241
[Non-Patent Document 3] Carbohydrate Research (2012) Vol. 348, p. 33-41
[Non-Patent Document 4] Journal of Oleo Science (2008) Vol.57, p.359-369

### SUMMARY OF THE INVENTION

As the conventionally-known structure of a sophorolipid, there are only about two structures, that is, the aforesaid lactonic form and acidic form. There is therefore a demand for increasing the variety of the structure for enhancing the physiological properties and the function of it.

An object of the present invention is to provide a novel sophorolipid derivative usable in a wide range of fields such as feedstuff, fertilizers, food and drink, agrichemicals, pharmaceuticals, quasi drugs, or cosmetics.

Another object of the present invention is to provide a composition containing a novel sophorolipid, particularly, a surfactant, a detergent, or an emulsifier containing it.

As a result of carrying out various instrumental analysis to find the presence of unknown components different in molecular structure from a conventionally known sophorolipid in a cultured product of a sophorolipid-producing microorganism which is widely studied, such as *Starmerella bombicola* , isolating and purifying them, and analyzing their structures, the present inventors have revealed that they are sophorolipid derivatives having a novel structure.

Finding based on the analysis of physical properties that these novel sophorolipid derivatives exhibit surface activity and self-organization characteristics different from those of the conventional sophorolipids and function as an excellent detergent component, they have completed the present invention.

The present invention relates to a sophorolipid derivative described in the following general (1) and (2).
(1) A sophorolipid derivative represented by the following general formula (1): 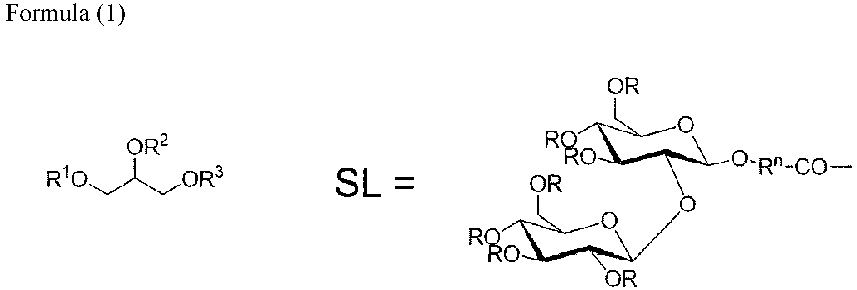 (wherein, R¹ to R³ are each independently hydrogen, a fatty acid ester having 2 to 22 carbon atoms, or the above SL group, provided that at least one of R¹ to R³ is the SL group in which R are the same or different and each represent hydrogen or an acetyl group and Rⁿ is a linear or branched alkyl or alkenyl group having 13 to 21 carbon atoms).
(2) A sophorolipid derivative which is any of compounds represented by the following chemical formula (2): 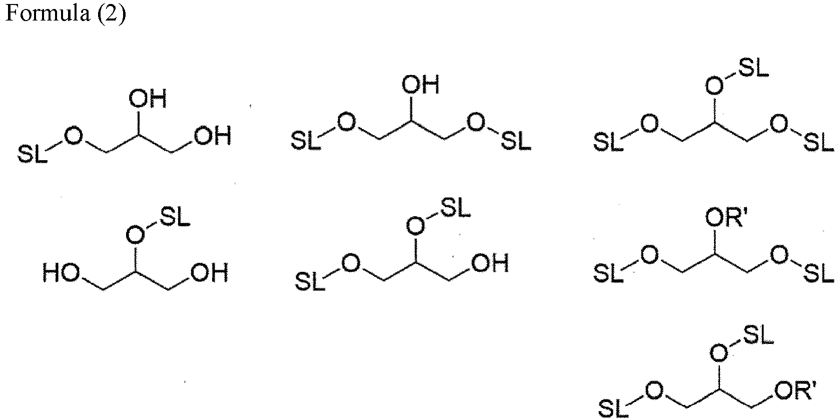 (wherein, the SL group has the same definition as in the above formula (1) and R' is a fatty acid ester having 2 to 22 carbon atoms).

Also, the present invention relates to a surfactant, a detergent, a dispersant, or an emulsifier described in the following (3) and (4).

(3) A surfactant, a detergent, a dispersant, or an emulsifier, comprising the sophorolipid derivative as claimed in the (1) or (2).

(4) The surfactant, the detergent, the dispersant, or the emulsifier according to the (3), which is feedstuff, a fertilizer, food and drink, an agrichemical, a pharmaceutical, a quasi-drug, or a cosmetic.

The novel sophorolipid derivatives of the present invention exhibit surface activity and self-organization characteristics different from those of conventional sophorolipids and function as an excellent detergent component, so that they can increase the structural and functional varieties of sophorolipid products. Compared to conventional sophorolipids, they function as a nonionic surfactant having high water solubility.

In addition, they are sophorolipid derivatives derived from highly-safe natural products, so that products obtained therefrom can have enhanced safety and therefore can be applied to a wide range of fields such as feedstuff, fertilizers, food and drink, agrichemicals, pharmaceuticals, quasi drugs, or cosmetics.

### BRIEF DESCRIPTION OF THE DRAWINGS

FIG. 1 shows the normal-phase TLC analysis results of a solid SL mixture obtained in Example 1;
FIG. 2 shows the reversed-phase TLC analysis results of the solid SL mixture obtained in Example 1;
FIG. 3 is a chromatogram showing the results of the reversed-phase column chromatography of the solid SL mixture obtained in Example 1;
FIG. 4 shows the LC/MS analysis results of Compound A separated in Example 4;
FIG. 5 shows the LC/MS analysis results of Compound B separated in Example 4;
FIG. 6 shows the LC/MS analysis results of Compound C separated in Example 4;
FIG. 7 shows the ¹H-NMR results of Compound B;
FIG. 8 shows the ¹H-NMR results (enlarged) of Compound B;
FIG. 9 shows the ¹H-NMR results of Compound A;
FIG. 10 shows the ¹H-NMR results of Compound C;
FIG. 11 is a photograph of 0.1 wt% aqueous solutions obtained by adding distilled water to Compounds A, B, and C, respectively;
FIG. 12 shows the surface tension reducing ability of each of Compounds A, B, and C, wherein A is indicated by pale ●, B by ∘, and C by ●;
FIG. 13 shows the MALDI-TOF/MS analysis results of the solid SL mixture obtained in Example 1;
FIG. 14 shows the results of HPLC analysis before and after the acetylation reaction of compound B; and
FIG. 15 shows the results of LC/MS analysis before and after the acetylation reaction of compound B.

### DESCRIPTION OF THE PREFERRED EMBODIMENTS

The present invention relates to a novel sophorolipid derivative and a composition containing the derivative.

The sophorolipid derivative (which may hereinafter be called "SL derivative") of the present invention is represented by the following general formula (1).

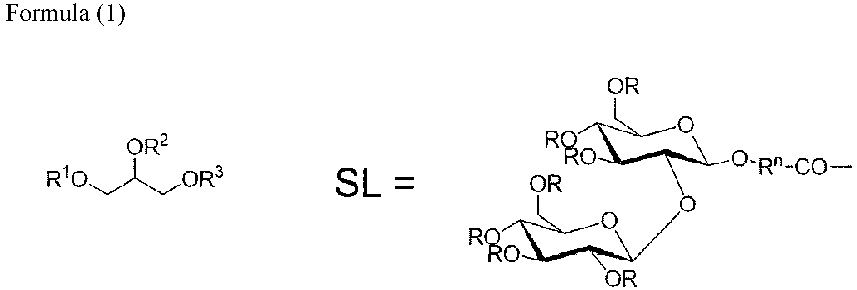

In the formula, R¹ to R³ each independently represent hydrogen, any of fatty acid esters having 2 to 22 carbon atoms, or the above SL group, provided that at least one of R¹ to R³ is the SL group.

Any of fatty acid esters having 2 to 22 carbon atoms is represented by -(O)C-hydrocarbon group and this hydrocarbon group has 1 to 21 carbon atoms.

R are the same or different and each represent hydrogen or an acetyl group.

Rⁿ is a linear or branched alkyl or alkenyl group having 13 to 21 carbon atoms.

The SL derivative of the present invention may be any one of the compounds represented by the following chemical formula (2):

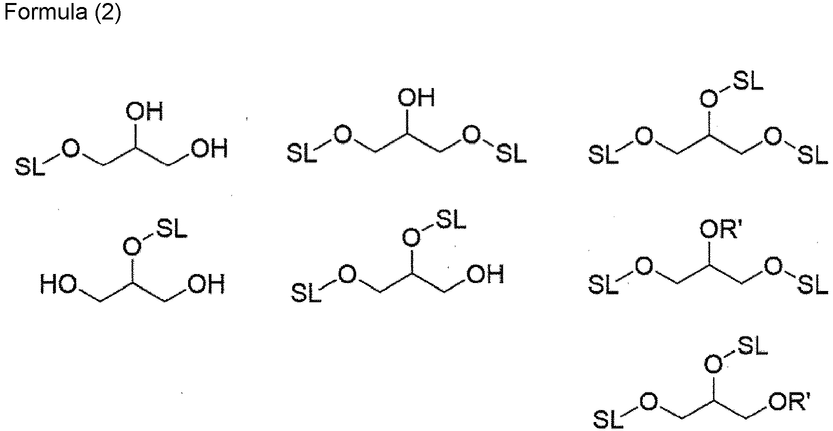

In the formula, the SL group has the same meaning as described in the formula (1) and R' is a fatty acid ester having 2 to 22 carbon atoms and is represented by a - (O)C- hydrocarbon group. The number of carbon atoms of this hydrocarbon group is 1 to 21.

The SL derivative of the present invention can be obtained from a cultured product of an SL-producing microorganism, but can be chemically synthesized because it is a glyceride of an SL.

For example, it can be produced by carrying out esterification (ester exchange or reverse reaction of hydrolysis) of a SL and glycerin with an immobilized enzyme such as lipase as a catalyst in a non-alcoholic organic solvent (chloroform, toluene, acetone, or the like) or without a solvent; or by carrying out an ester exchange reaction using a plant oil (triglyceride) instead of the glycerin. In particular, when a lactonic form (LSL) having excellent reactivity is used, the SL derivative of the present invention can be produced by mixing it with glycerin or the like and heating and stirring the resulting mixture.

Furthermore, a hydroxyl group of a SL group can be acetylated by reacting it with acetic anhydride or the like even after it is obtained from the culture. By utilizing this reaction, it is possible to produce a SL derivative (for example, all Rs of the SL groups of chemical formula (1) are acetyl groups) with improved lipophilicity by introducing acetyl groups to all hydroxyl groups.

As a surfactant, an emulsifier, or a dispersant, depending on the surface activity of the SL derivative of the present invention, the composition of the present invention can be applied to feedstuff, fertilizers, food and drink, agrichemicals, pharmaceuticals, quasi drugs, or cosmetics, or additives thereto.

Although an amount of the SL derivative contained in the composition of the present invention is not particularly limited, it is 0.01 to 100 wt%, preferably 0.1 to 50 wt%, more preferably 1 to 30 wt%. When the amount of the SL derivative in the composition is as small as 1 wt% or less, the resulting composition has less water solubility, while the amount as large as 50 wt% or more may reduce the economic efficiency.

### [Examples]

The present invention will hereinafter be described more specifically by Examples but the present invention will not be limited to or by the following Examples. In Examples, "%" means "wt%".

### [Example 1]

### Production and recovery of sophorolipid

### Culture of Starmerella bombicola ATCC A22214 strain

### (1) Seed culture

A platinum loopful of the aforesaid *Starmerella bombicola* ATCC 22214 strain which had been kept in a preservation medium (10 g/L of a yeast extract, 20 g/L of peptone, 20 g/L of glucose, and 20 g/L of agar) was inoculated in a test tube containing 4 mL of a liquid medium composed of 10 g/L a yeast extract, 20 g/L of peptone, and 20 g/L of glucose and shake-cultured at 28°C for one day.

### (2) Main culture

The microbial culture solution obtained in (1) was inoculated in an Erlenmeyer flask containing 30 mL of a liquid medium composed of 10 g/L of a yeast extract, 20 g/L of peptone, 100 g/L of glucose, and 100 g/L of a rapeseed oil and shake-cultured at 28°C for 7 days.

### (3) Recovery of sophorolipid

By allowing the culture solution obtained in (2) to stand for one hour, an SL phase appeared in the lower layer. The resulting SL phase was recovered, neutralized with an aqueous solution of sodium hydroxide, and washed with water to recover the SL mixture in the form of an aqueous solution. In order to perform a test later on the resulting aqueous solution of the SL mixture, a solid SL mixture was recovered by adding hexane to the aqueous solution, stirring and centrifuging the resulting mixture to extract and remove the remaining oil or fat, or fatty acid in the hexane phase, and freeze drying the aqueous phase.

### (4) Purification of lactonic sophorolipid and acidic sophorolipid specimens

To use the resulting mixture solid as a specimen in the subsequent experiment, a lactonic SL (LSL) and an acidic SL (ASL) were separated from each other by a known purification technique. Described specifically, the solid SL mixture was dissolved in acetone and the resulting solution was placed on a silica gel column obtained by filling a glass column tube with silica gel (Wakogel C-200) and purified by column chromatography using a chloroform-acetone mixture as a developing solvent. The separation and recovery of LSL were performed at a chloroform:acetone ratio of 8:2, followed by those of acidic SL at 2:8. The fractions thus recovered were confirmed to be intended LSL specimen and ASL specimen, respectively, by thin-layer chromatographic analysis which will be described later.

### [Example 2]

Thin-layer chromatographic analysis of sophorolipid

The solid SL mixture obtained in Example 1 was dissolved in methanol and the resulting solution was subjected to thin-layer chromatographic (TLC) analysis.

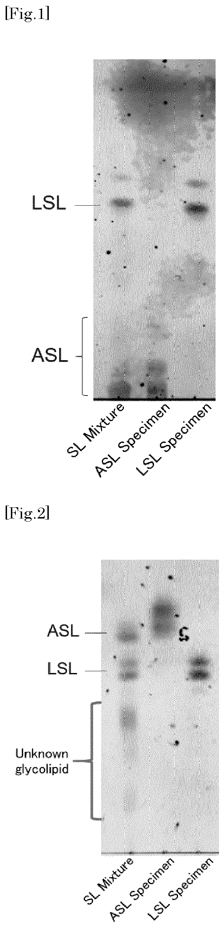

First, conventional normal-phase TLC analysis using a chloroform/methanol/aqueous ammonia = 80/20/2 mixed solvent as a developing solvent was performed on a silica gel TLC glass plate (Silica gel 60F254 glass plate, product of Merck). When the component is detected using an anthrone-sulfuric acid indicator, a compound containing a sugar skeleton is detected as a bluish green color. As a result, non-polarity LSL was developed in the upper phase, while a high-polarity ASL was developed in the lower phase (FIG. 1).

Next, reversed-phase analysis using a methanol/water = 95/5 mixed solvent as a developing solvent was performed on a reversed-phase modified silica gel TLC plate (TLC silica gel 60RP-18F254S glass plate, product of Merck). As expected, contrary to the aforesaid result in the normal phase, high-polarity ASL was developed in the upper phase. Further, non-polarity LSL was developed in the intermediate phase and a spot presumed to be derived from a structurally unknown glycolipid not detected by the conventional method was detected in the lower phase (FIG. 2).

The results have revealed that the SL mixture obtained in Example 1 contains a glycolipid different from the conventionally known LSL and ASL.

### [Example 3]

### High-performance liquid chromatographic analysis of sophorolipid

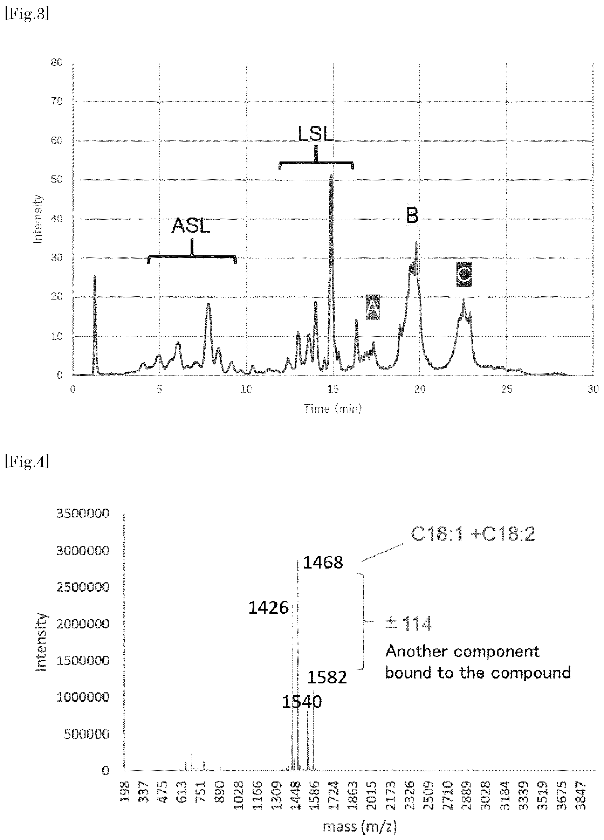

The solid SL mixture obtained in Example 1 was dissolved in methanol and the resulting solution was subjected to high-performance liquid chromatographic (HPLC) analysis. Component analysis was performed using HPLC (Thermo Scientific Ultimate 3000 HPLC, product of Thermo Fisher Scientific) equipped with a corona charged aerosol detector (CAD) (Corona^{™} Veo^{™}, product of Thermo Fisher Scientific); using a reversed-phase ODS column (InertSustain^{™} VC18, product of GL Science) as an analysis column; at a column temperature of 40°C; using a (5 mM ammonium formate methanol)/(5 mM aqueous ammonium formate) mixed solvent system as an eluting solution; according to the following gradient program: 70% methanol (step: 0 min to 3 min), 70% to 100% (gradient: 3 min to 18 min), 100% (step: 18 min to 25 min), and 70% (step: 25 min to 35 min); and at a flow rate of 0.3 mL/min. The chromatogram thus obtained is shown in FIG. 3.

As a result of the analysis, ASL and LSL were detected after the retention time of 3 to 10 minutes and 12 to 16 minutes, respectively, and then, three unknown component peaks (A, B, and C) were detected after around 18 minutes, around 20 minutes, and around 22 minutes, respectively. The results were consistent with those of the reversed-phase TLC analysis in Example 2 showing the detection of an unknown glycolipid after that of ASL and LSL.

### [Example 4]

### Separation/purification of structurally-unknown glycolipid

To isolate the three peak components newly detected in Example 3, the components were separated from each other by silica gel column chromatography. First, almost all the amount of the LSL fraction was eluted with a chloroform/acetone = 50/50 solvent by the normal-phase column method using a chloroform/acetone mixed solvent similar to that used in Example 1 (4) and then, a remaining component containing a mixture of ASL and the intended unknown glycolipids was recovered with a solvent composed of 100% acetone or 100% methanol. Next, by the reversed-phase column chromatography with a methanol/water mixed solvent as a developing solvent on a column filled with octadecyl-modified silica gel (Wakogel 100C18), the unknown glycolipids were separated/recovered using a two-step column purification method for separating ASL from the unknown glycolipids. Each of the components thus obtained by separation was confirmed to be almost a single-spot component and a single-peak component by the reversed-phase TLC analysis and the reversed-phase HPLC analysis, respectively.

The components will hereinafter be called "Compound A", "Compound B", and "Compound C" in the order in which they were eluted from the reversed-phase column. Compound A was a clear and viscous solid, B was a clear solid harder than A, and C was a waxy white solid.

### [Example 5]

### Mass spectrum analysis of structurally unknown glycolipids

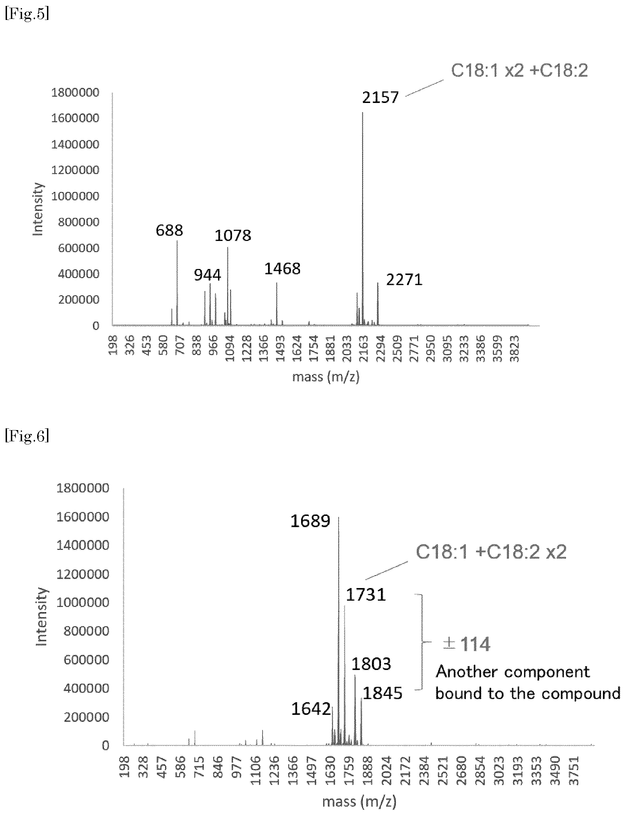

Compounds A, B, and C separated from each other in Example 4 were subjected to liquid chromatography/mass spectrum (LC/MS) analysis. FIGS. 4 to 6 show the respective results of the LC/MS analysis conducted under separation conditions similar to those of Example 3 by using the HPLC described in Example 3 to which an electrospray ionization mass spectrometer (ESI-MS) (Exactive Plus, product of Thermo Fisher Scientific) was connected.

Compounds A, B, and C had, as a main component thereof, m/z=1468, m/z=2157, and m/z=1731 (each, detection in negative mode) and contained components (1426, 2115, and 1689) obtained by subtracting 42 from the main components, respectively. Such a pattern was frequently observed from glycolipid type biosurfactants and the component other than the main component was presumed to have one acetyl group removed therefrom. Further, they contained components (1582, 2271, and 1845) obtained by adding 114 to the main components or components obtained by subtracting 42 therefrom, respectively. The components were all presumed to be obtained by partially modifying the functional group of the main component. Compounds A to C are presumed to contain components having the same basic structure but different in fatty acid composition.

### [Example 6]

Structural analysis of structurally-unknown glycolipids

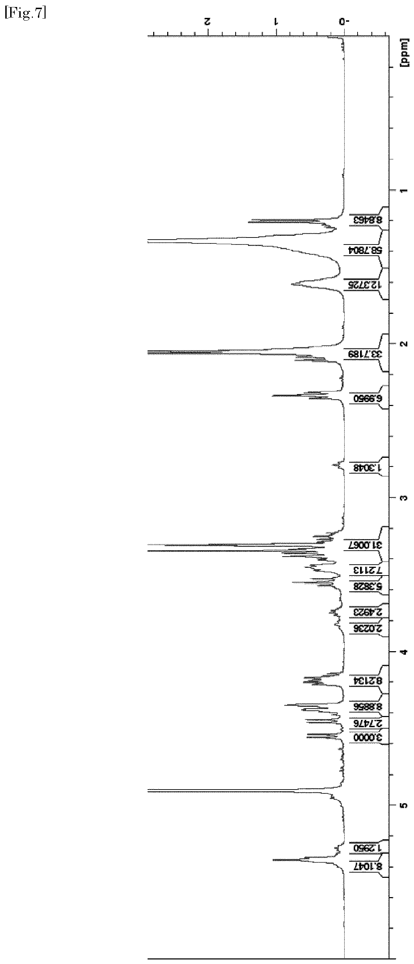

The chemical structure of Compounds A, B, and C separated from each other in Example 4 was identified by the nuclear magnetic resonance spectrum (NMR) analysis. It has been confirmed that as a result of the ¹H-NMR analysis of Compound B showing the largest content among the three components by NMR (AV-400), product of Bruker, the spectrum is almost the same as that of the structurally known ASL (FIG. 7).

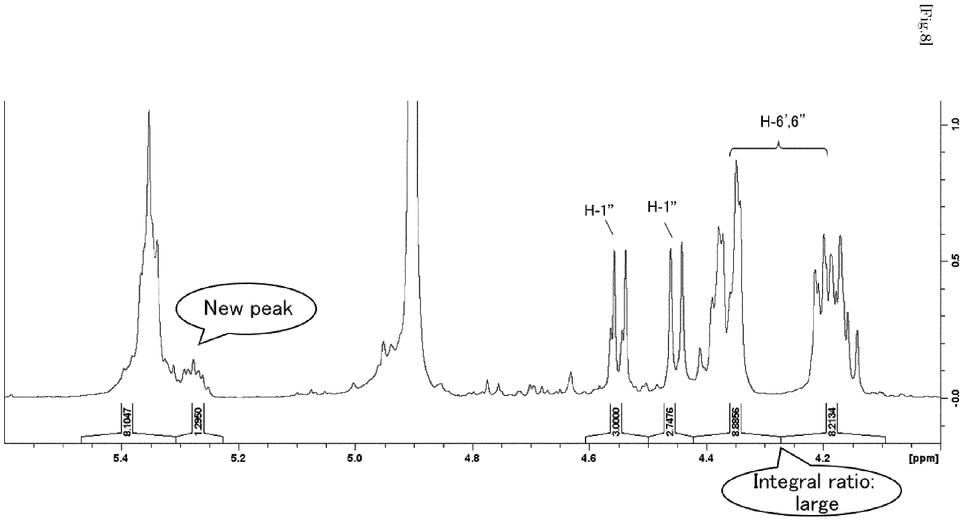

On the other hand, the spectrum of the sugar skeleton portion was enlarged and compared. As a result, it has been confirmed that a new peak different from that of ASL appeared near 5.3 ppm and a peak near 4.1 to 4.4 ppm had a peak area ratio obviously larger than that of the peak at the 1 position of sophorose as the referential peak. To specifically analyze these peaks further, ¹H-¹HCOSY analysis was performed to find that a new peak different from a peak which might originally appear near 4.1 to 4.4 ppm and was derived from the 6 position of sophorose appeared and they overlapped in position, showing the correlation with the new peak near 5.3 ppm (FIG. 8).

The NMR data of Compound B are shown in Table 1.

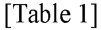

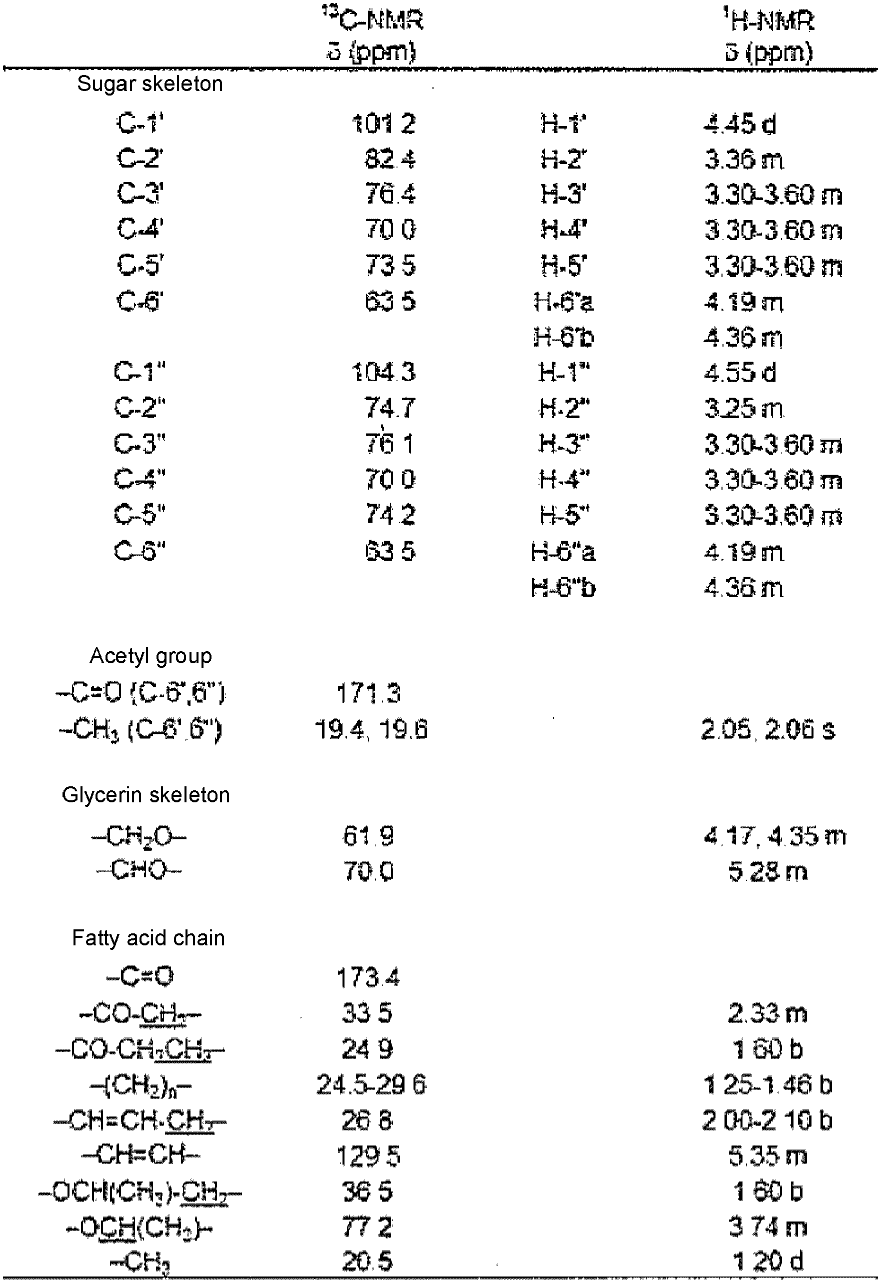

It has been found from the aforesaid results that Compound B is an SL derivative having, in addition to the structure of ASL, a chemical structure showing those two kinds of peaks. Examples of a typical compound exhibiting peaks near 5.3 ppm and near 4.1 to 4.4 ppm include the glycerin skeleton of an oil or fat (fatty acid triglyceride). Based on the aforesaid finding, Compound B is presumed to be represented by the chemical formula (6) in which ASL is ester-linked to three hydroxyl groups of glycerin.

The main component of Compound B confirmed in Example 5 was m/z=2157, which was completely consistent with the molecular weight of a compound having a triglyceride structure in which two ASLs having a C18:1 fatty acid site and one ASL having a C18:2 fatty acid site were ester linked to glycerin. Based on the above findings, Compound B is identified as the compound of the chemical formula (6).

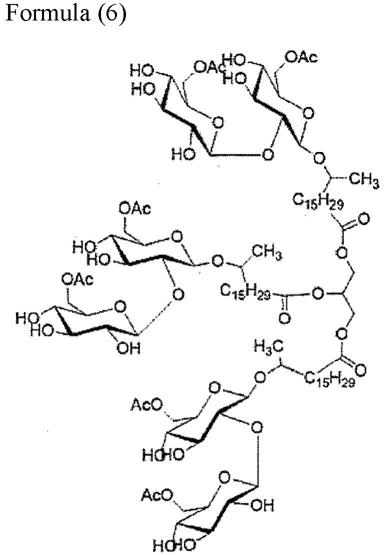

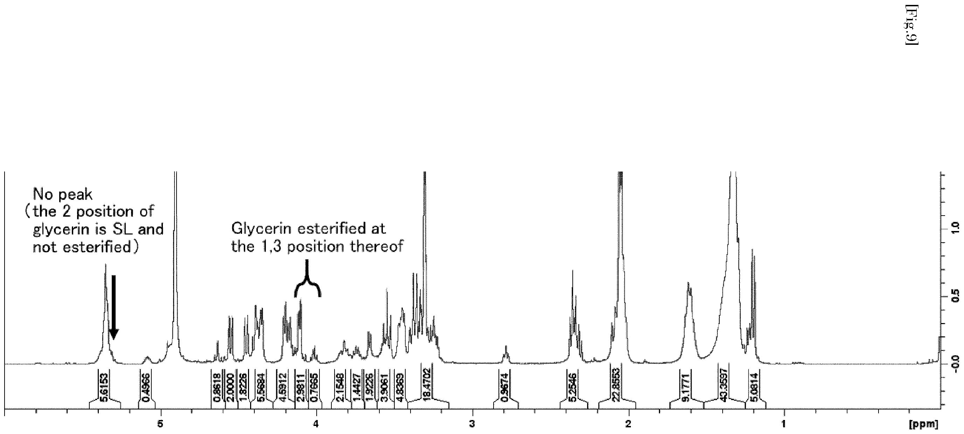

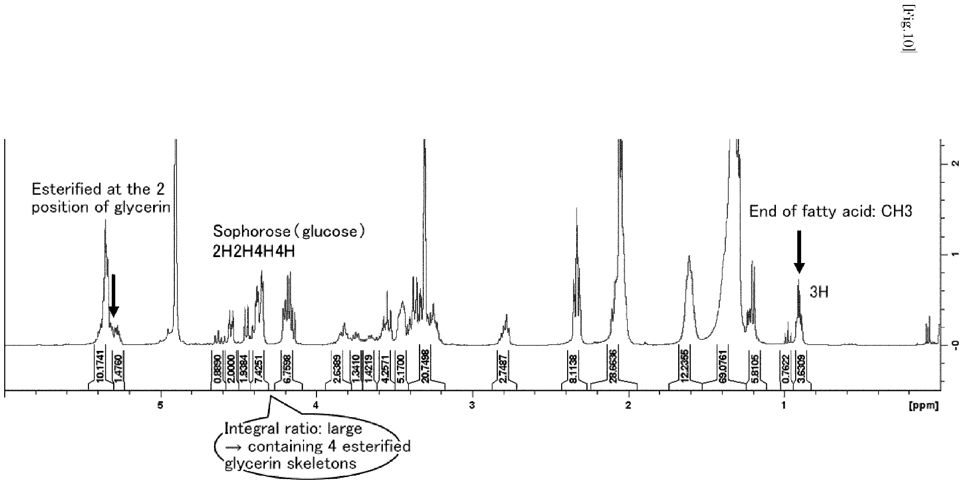

Compounds A and C were subjected to similar analysis. The results of the ¹H-NMR analysis of Compounds A and C are shown in FIGS. 9 and 10. The NMR data of Compound A or C are shown in Table 2 or 3.

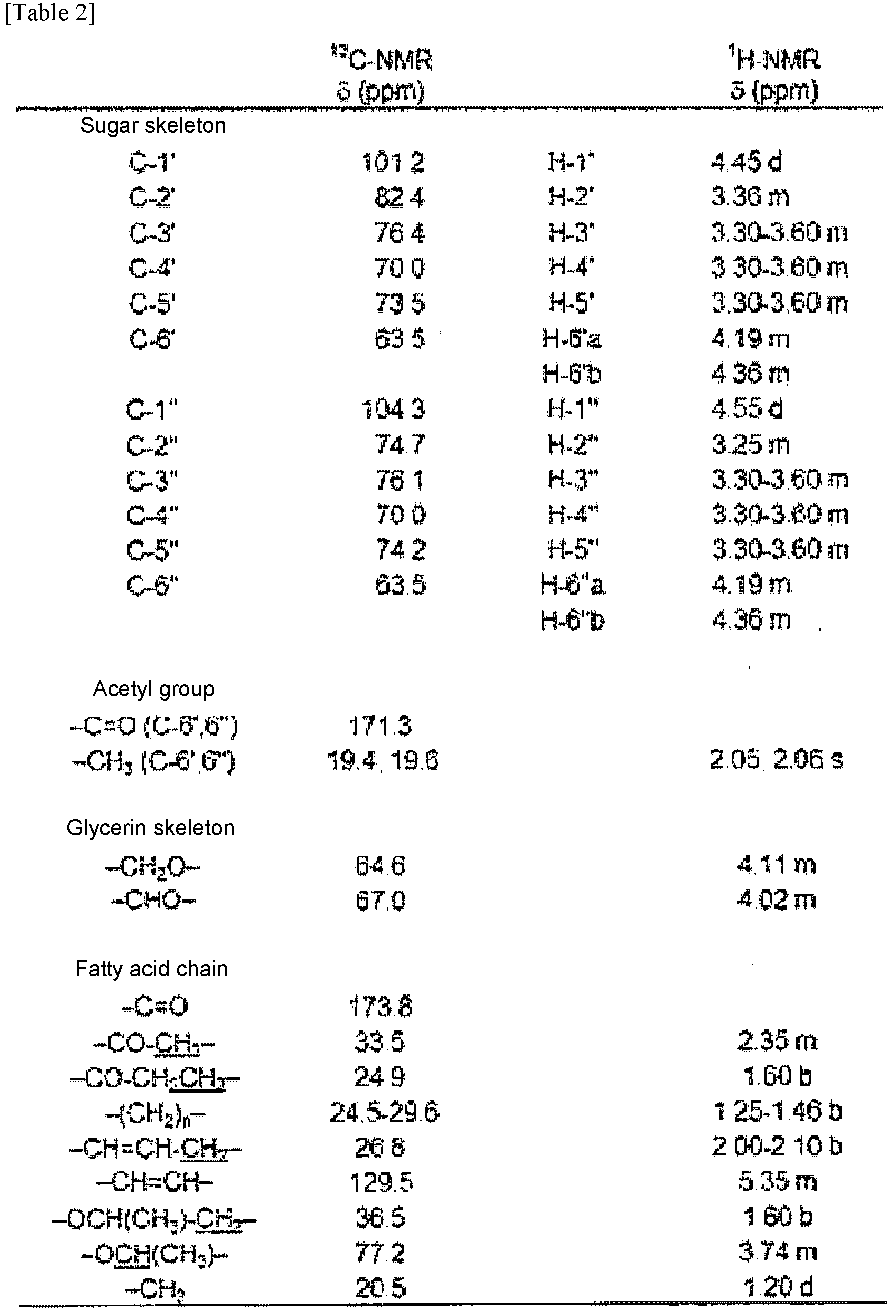

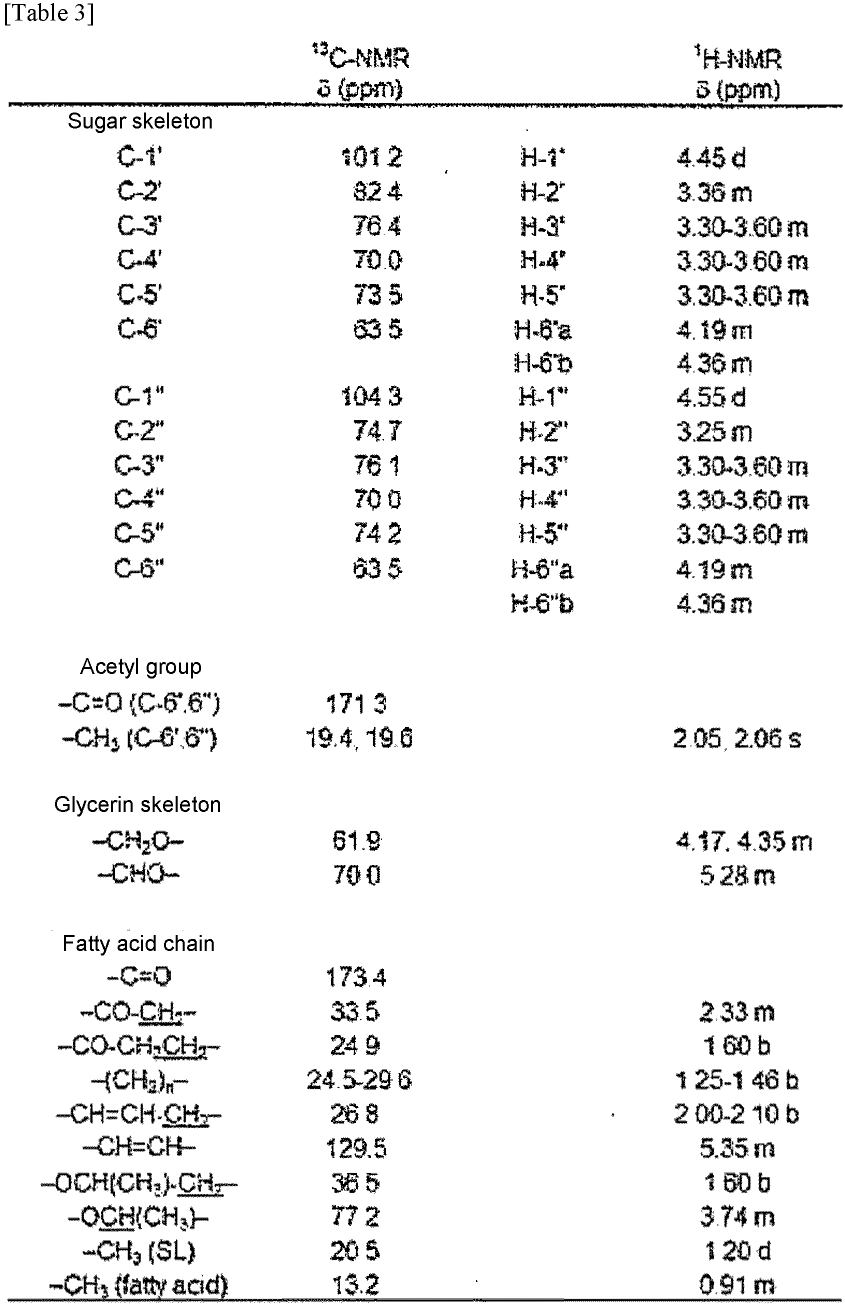

Based on the NMR measurement results, as a most likely compound having a representative structure, Compound A is presumed to be a compound of the chemical formula (5) in which two ASLs are linked to glycerin and Compound C is presumed to be a compound of the chemical formula (7) in which two ASLs and one long-chain fatty acid are ester-linked to glycerin. Their molecular weight is consistent with that of the main component of each compound confirmed in Example 5, supporting the results of this structural analysis.

Derivatives contained in the cultured product of a producing microorganism are not limited to them.

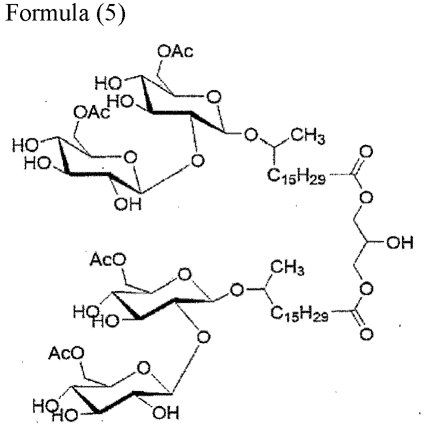

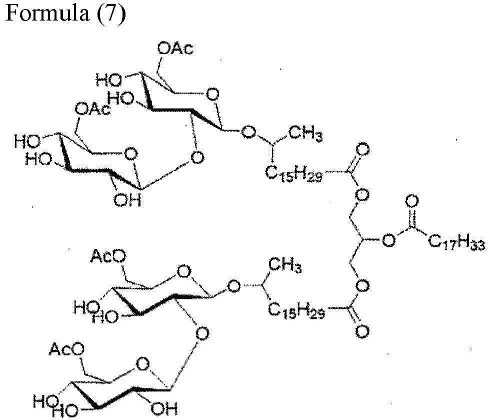

### [Example 7]

### Aqueous solutions of novel sophorolipid derivatives A to C

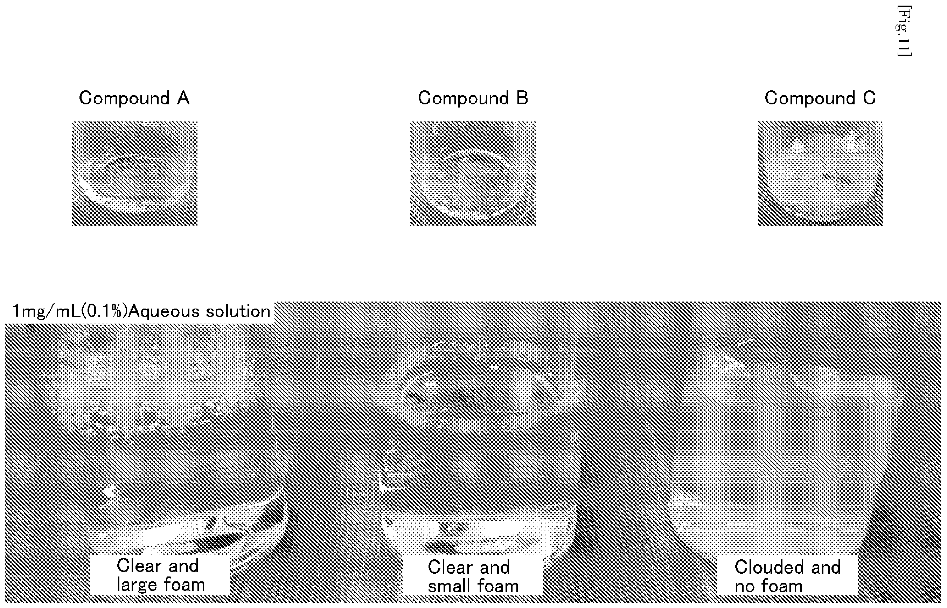

Compounds A, B, and C separated from each other in Example 4 were weighed (each, 10 mg) in measuring flasks, respectively, and diluted with distilled water to prepare 1 mg/mL (0.1 wt.%) aqueous solutions (FIG. 11).

Clear aqueous solutions were obtained by completely dissolving Compound A and Compound B, but the aqueous solution of Compound C became clouded. Further, 10 mg/mL (1 wt%) aqueous solutions of Compounds A and B were prepared respectively in a similar manner. The aqueous solution of A became clouded and Compound B was dissolved completely. The clouded aqueous solutions of Compounds A and C were allowed to stand in a refrigerator and then, they were both dissolved completely to be a clear aqueous solution.

The phase change of the aqueous solutions depending on the temperature is a reversible phenomenon, showing that it has a cloud point at room temperature (25°C) or less. This supports that Compounds A and C are a nonionic surfactant as structurally determined in Example 6. Further, the above results show that compared to Compound C having a long-chain fatty acid bonded thereto, Compound A not having it shows higher water solubility and Compound B is anionic surfactant having markedly high water solubility, though having a higher molecular weight than the other two.

### [Example 8]

### Surface tension reducing ability of novel SL derivatives A to C

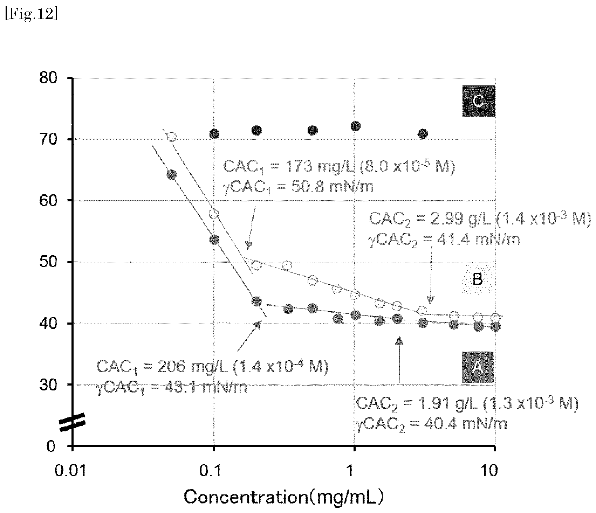

Aqueous solutions of Compounds A, B, and C separated from each other in Example 4 and having various concentrations were prepared and the surface tension of them was determined by the pendant drop method (Young Laplace method) using a contact angle meter (DMo-500, product of Kyowa Interface Science). FIG. 12 shows a logarithmically plotted graph of the concentration and the surface tension of the aqueous solutions.

Compound C showed almost no change in the surface tension of its aqueous solution regardless of the concentration, while Compounds A and B showed a significant reduction in surface tension with an increase in the concentration. In addition, both were confirmed to have two-step inflection points in the plot with an increase in the concentration. When a critical micelle concentration (CMC) was calculated from the latter inflection point from which the surface tension became constant, Compound A had CMC of 1.91 g/L (about 1.3 × 10⁻³ M) and had a surface tension (yCMC) of 40.4 mN/m at that concentration and Compound B had CMC of 2.99 g/L (about 1.4 × 10⁻³ M) and had γCMC of 41.4 mN/m. With respect to LSL and ASL, LSL has CMC of 1.4 × 10⁻⁵ M and γCMC of 32.3 mN/m and ASL has CMC of 1.2 × 10⁻⁴ M and γCMC of 37.1 mN/m, according to the literature. Compared to the conventional compounds, the novel SL derivatives found this time have remarkably high water solubility and exhibit mild surface tension reducing ability, though they are a nonionic surfactant, in other words, they are a surfactant very suitable for use in an aqueous system.

### [Example 9]

### Matrix assisted laser ionization time-of-flight mass spectrum (MALDI-TOF/MS) analysis of sophorolipid

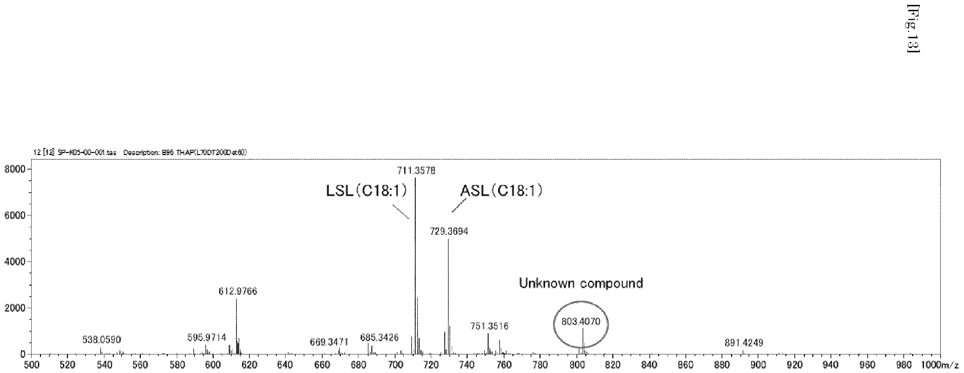

MALDI-TOF/MS analysis of the solid SL mixture obtained in Example 1 was performed by JMS-3000 SpiralTOF-MS, product of JASCO Corporation, with 2',4',6'-trihydroxyacetophenone (THAP) as a matrix (FIG. 13).

A peak of a structurally-unidentified compound having a structure of m/z=803.4 and different from that of conventional LSL and ASL was detected. Based on the molecular weight calculated with reference to the structure of Compounds A to C in which SL is ester-linked to glycerin, it is presumed to be a compound (Na adduct) in which one ASL having a C18:1 fatty acid site is ester-linked to glycerin as shown in the chemical formula (8).

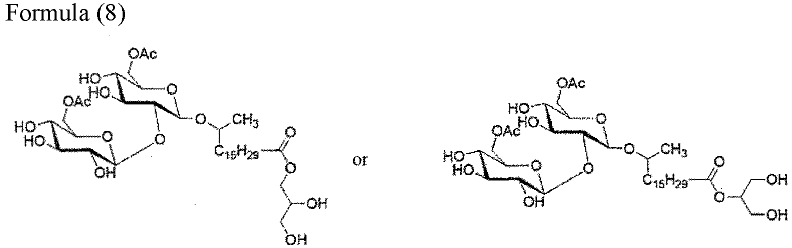

### [Example 10]

### Acetylation of sophorolipid derivatives

The compound B obtained in Example 4 (20 mg) was dissolved in acetone (1 mL) in a vial bottle, and acetic anhydride (200 µL) and triethylamine (10 µL) were added, respectively. The reaction solution was allowed to stand overnight at room temperature. After the reaction, all solvents were removed by drying under reduced pressure, and the product was recovered.

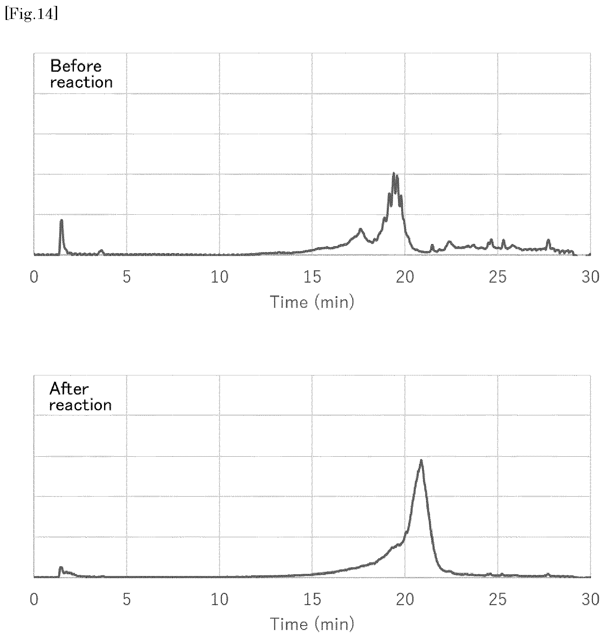

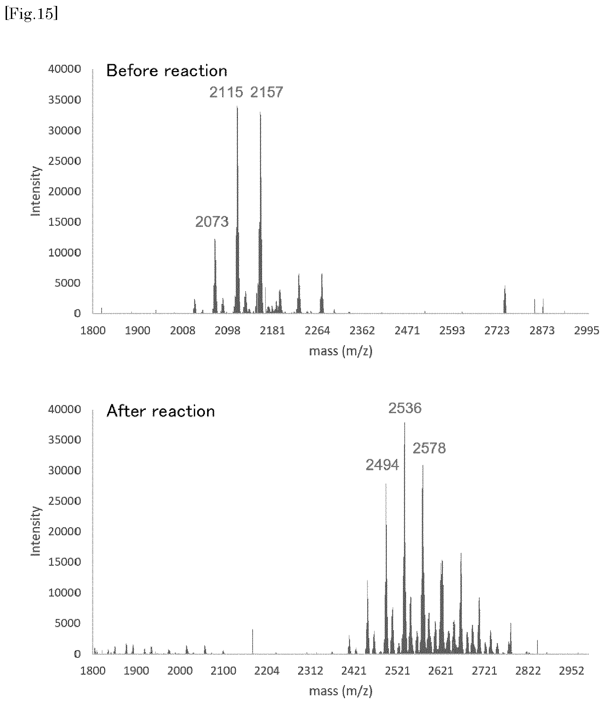

Reversed-phase TLC analysis of the product detected a compound with a lower mobility due to the acetylation, that is more hydrophobic than the starting compound B. Therefore, the progress of the acetylation reaction was confirmed. Subsequently, HPLC and LC/MS analysis of the product was performed in the same method as in Examples 3 and 5. On the HPLC chart, the peak of the product shifted to the longer retention time side, that is, to the hydrophobic side (FIG. 14), as in the above reversed-phase TLC. In addition, the molecular ion peaks of the main component of the product were 2536, 2578, 2494, etc. These were confirmed to be acetylated products in which 9, 10, and 8 acetyl groups are bonded to the sugar moiety of the compound B, respectively (FIG. 15). ¹H NMR analysis of the product also supported that the acetyl group was additionally introduced into the compound B due to the increase of the peak derived from the acetyl group at around 2.0 ppm.

By using a similar method, it is possible to introduce any number of acetyl groups into the compounds A and C as well. From the above results, it was confirmed that the SL derivatives of the present invention can be converted into novel SL derivatives with any number of acetyl groups (Formula (1)) by an acetylation reaction.

### Industrial Availability

The novel SL derivatives of the present invention are sophorolipid derivatives having excellent water solubility, surface tension reducing ability, and self-organization characteristics and derived from highly-safe natural products, so that they can be applied to a wide range of fields such as feedstuff, fertilizers, food and drink, agrichemicals, pharmaceuticals, quasi drugs, and cosmetics.

## Claims

1. A sophorolipid derivative represented by the following general formula (1): 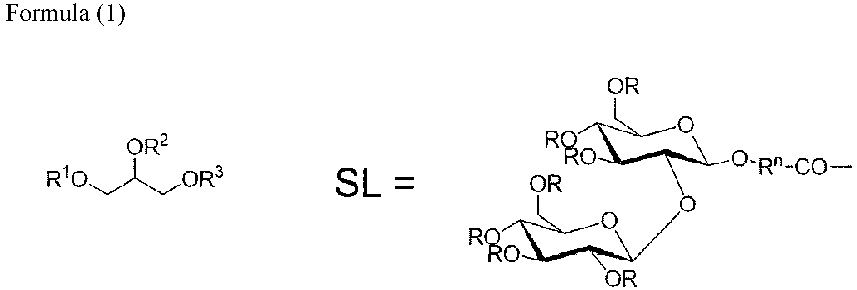 (wherein, R¹ to R³ are each independently hydrogen, a fatty acid ester having 2 to 22 carbon atoms, or the above SL group, provided that at least one of R¹ to R³ is the SL group in which R are the same or different and each represent hydrogen or an acetyl group and Rⁿ is a linear or branched alkyl or alkenyl group having 13 to 21 carbon atoms).

2. A sophorolipid derivative which is any of compounds represented by the following chemical formula (2): 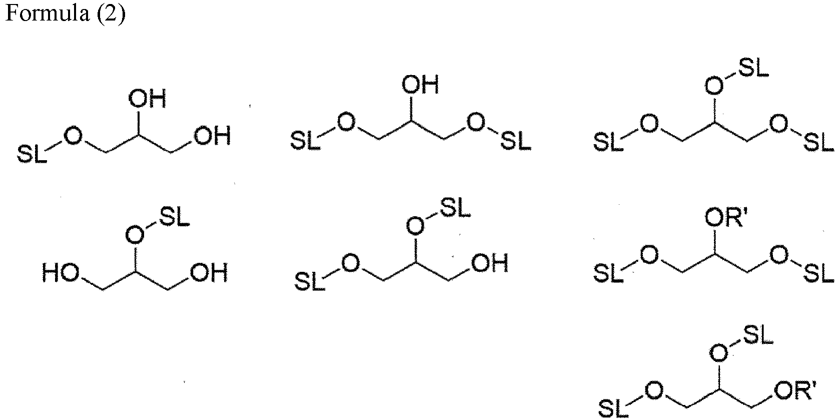 (wherein, the SL group has the same definition as in the above formula (1) and R' is a fatty acid ester having 2 to 22 carbon atoms).

3. A surfactant, a detergent, a dispersant, or an emulsifier, comprising the sophorolipid derivative as claimed in Claim 1 or 2.

4. The surfactant, the detergent, the dispersant, or the emulsifier according to Claim 3, which is feedstuff, a fertilizer, food and drink, an agrichemical, a pharmaceutical, a quasi-drug, or a cosmetic.
